# FASCICULE DE BREVET EUROPEEN

(11) **EP 1 077 687 B1**
(45) Date de publication et mention de la délivrance du brevet: **20.03.2002**
(21) Numéro de dépôt: 99920897.8
(22) Date de dépôt: 20.05.1999
(51) Int. Cl.: A61K 9/54

(54) **FORME PHARMACEUTIQUE MULTIPARTICULAIRE A LIBERATION PROGRAMMEE ET PULSEE ET SON PROCEDE DE PREPARATION**
MULTIPARTIKULÄRE PHARMAZEUTISCHE FORM MIT GESTEUERTER UND GEPULSTER WIRKSTOFFABGABE UND VERFAHREN ZU DEREN HERSTELLUNG
MULTIPARTICULATE PHARMACEUTICAL FORM WITH PROGRAMMED AND TIMED RELEASE AND PREPARATION METHOD

(30) Priorité: 20.05.1998 FR 9806384
(43) Date de publication de la demande: 28.02.2001
(73) Titulaire: LABORATOIRES PROGRAPHARM, 28170 Châteauneuf-en-Thymerais (FR)
(72) Inventeur: GENDROT, Edouard, F-28500 Garnay (FR); COUSIN, Gérard, F-28210 Le Mesnil Ponceau (FR); RAGOT, Françoise, F-28300 Lèves (FR); CLEE-BOUVET, Marie-Christine, F-28500 Treon (FR)
(74) Mandataire: Touati, Catherine
(86) Numéro de dépôt international: FR9901201
(87) Numéro de publication internationale: WO9959557

(56) Documents cités:
- EP-A- 0 463 877
- WO-A-98/17261
- US-A- 5 567 441

## Description

L'invention a pour objet une forme pharmaceutique multiparticulaire à libération programmée et pulsée pour administration orale. Elle a également pour objet le procédé de préparation de cette forme pharmaceutique.

De nombreuses formes pharmaceutiques à libération prolongée pour administration orale existent sur le marché. La libération du principe actif doit être contrôlée en fonction de l'objectif thérapeutique et des propriétés pharmacologiques du principe actif. Il n'est donc pas toujours souhaitable que le taux plasmatique soit constant. Au contraire, pour éviter toute accoutumance et pour limiter les effets secondaires provoqués par le principe actif, il serait tout à fait avantageux que le taux plasmatique suive le rythme métabolique et les besoins spécifiques du patient à certaines périodes du nycthémère. Par exemple, afin de diminuer les symptômes nocturnes ou du réveil de certaines maladies chroniques telles que l'ischémie cardiaque, l'asthme et l'arthrite, les médicaments devraient être administrés de façon à ce que le niveau plasmatique thérapeutique souhaité ne soit atteint qu'au moment requis, c'est-à-dire durant le sommeil ou juste au moment du réveil.

Dans l'article "An Organic Acid-Induced Sigmoidal Release System for Oral Controlled-Release Preparations" ("Système à Libération Sigmoïdale Induite par Acide Organique pour des Préparations Orales à Libération Contrôlée"), Pharmaceutical Research, Vol.11, N°1 1994, est décrite une forme pharmaceutique à libération de type sigmoïdal. Cependant, il est indiqué dans cet article que le type de libération sigmoïdal du principe actif ne peut être obtenu que si l'on ajoute dans la forme pharmaceutique un acide organique. Or, de par la présence d'un acide organique, de telles formes pharmaceutiques peuvent avoir un effet irritant, ce qui est un inconvénient majeur notamment lorsque les traitements sont de longue durée.

WO 98/17261 décrit une forme pharmaceutique à libération contrôlée comprenant deux types de sphéroïdes médicamenteux ainsi que son procédé de préparation.

A ce jour, il n'existe donc pas de forme pharmaceutique multiparticulaire exempte d'acide organique capable de mettre à disposition de fortes concentrations en principe actif au moment où le patient en a le plus besoin, tout en maintenant tout au long de la journée la concentration plasmatique thérapeutique minimale.

La Société Demanderesse a eu le mérite de trouver qu'il est possible d'obtenir de façon inattendue et surprenante de telles formes pharmaceutiques qui sont faciles à administrer et qui n'ont pas d'effet irritant.

Ainsi la forme pharmaceutique multiparticulaire selon l'invention qui est à libération retardée et pulsée et qui permet d'obtenir le début de la mise à disposition du principe actif dans les 4 à 8 heures suivant l'ingestion de la forme pharmaceutique, puis la libération progressive de la totalité du principe actif dans les 8 à 20 heures suivantes, est caractérisée par le fait qu'elle est exempte d'acide organique et qu'elle se présente sous la forme de microgranules ou sphéroïdes médicamenteux constitués d'un centre neutre comportant un premier enrobage à base d'un mélange d'au moins un polymère hydrosoluble et d'au moins un polymère non hydrosoluble au sein duquel sont uniformément réparties des particules constitutives d'un principe actif, l'ensemble comportant un deuxième enrobage à base d'au moins deux polymères pH indépendants, présentant des degrés de perméabilité vis-à-vis des milieux gastrique et intestinal différents, éventuellement d'au moins un polymère pH dépendant, d'au moins un plastifiant et d'au moins une charge inerte uniformément répartie dans ledit enrobage.

La concentration en principe actif est très élevée dans la couche entourant le support sphérique neutre, ce qui permet d'obtenir des sphéroïdes médicamenteux de dimension réduite et par là même des formes pharmaceutiques finales de petite taille qui sont plus faciles et plus agréables à ingérer par le patient et permettent d'administrer des dosages plus importants par unité de prise thérapeutique.

Selon un mode de réalisation avantageux, la forme pharmaceutique conforme à l'invention est donc caractérisée par le fait que le principe actif représente de 20 à 100 % en poids de la couche enrobant le support sphérique neutre, de préférence de 60 à 90 % en poids et plus préférentiellement encore de 80 à 90 % en poids.

Le polymère hydrosoluble entrant dans la composition de la couche enrobant le support sphérique neutre et contenant le principe actif est choisi dans le groupe comprenant notamment la polyvinylpyrrolidone, l'hydroxypropylméthyl cellulose et leurs mélanges, et le polymère non hydrosoluble est choisi dans le groupe comprenant notamment les résines acryliques et/ou méthacryliques, les polymères cellulosiques, et leurs mélanges.

Le rapport massique entre le polymère hydrosoluble et le polymère non hydrosoluble est de 0,1 à 0,9, de préférence de 0,3 à 0,7, et plus préférentiellement encore de 0,45 à 0,55.

Le deuxième enrobage de la forme pharmaceutique selon l'invention est à base d'au moins deux polymères pH indépendants, présentant des degrés de perméabilité vis-à-vis des milieux gastrique et intestinal différents, éventuellement d'au moins un polymère pH dépendant, d'au moins un plastifiant et d'au moins une charge inerte uniformément répartie dans ledit enrobage.

Dans ce deuxième enrobage, la proportion entre le polymère présentant la perméabilité la plus faible et le polymère présentant la perméabilité la plus grande est de 97/3 à 80/20.

Il convient de respecter ces proportions entre les polymères de degrés de perméabilité différents. En effet, pour des proportions en polymère de faible perméabilité supérieure à 97%, la libération du principe actif peut être trop retardée ; en revanche pour des proportions en polymère de faible perméabilité inférieures à 80%, la libération du principe actif n'est pas suffisamment retardée.

Les polymères entrant dans la constitution du deuxième enrobage sont choisis dans le groupe comprenant notamment les résines acryliques et/ou méthacryliques, les polymères cellulosiques, et leurs mélanges.

La charge inerte uniformément répartie dans le deuxième enrobage est choisie dans le groupe comprenant notamment le talc, la silice colloïdale anhydre, le stéarate de magnésium, le monostéarate de glycérol et leurs mélanges.

Le plastifiant entrant dans la constitution du deuxième enrobage doit être pharmaceutiquement acceptable; il est choisi dans le groupe comprenant notamment le phtalate d'éthyle, le triéthylcitrate, le dibutylsébacate, la triacétine et leurs mélanges.

Selon un mode de réalisation avantageux de l'invention, le principe actif est choisi dans le groupe comprenant notamment les molécules actives sur le système cardio-vasculaire, et plus particulièrement le Diltiazem et le Verapamil, les anti-inflammatoires, les antiallergiques et les antihistaminiques.

La forme pharmaceutique conforme à l'invention est administrée au moment du coucher.

Ainsi, la mise à disposition du principe actif débutera environ 4 à 8 heures après l'ingestion, c'est-à-dire au moment du réveil du patient, moment où certains symptômes dont les risques d'accidents cardiovasculaires sont rapportés être les plus importants.

La libération se poursuit régulièrement pendant les 8 à 20 heures suivantes.

L'administration de la forme pharmaceutique suivante étant effectuée quelques heures après la fin de la libération totale du principe actif, le patient bénéficie de la concentration plasmatique thérapeutique minimale sur l'ensemble du nycthémère.

Le procédé conforme à l'invention pour la préparation de la forme pharmaceutique conforme à l'invention est caractérisé par une succession d'étapes comprenant:
- l'introduction de supports sphériques neutres dans une enceinte réactionnelle à lit fluidisé,
- la pulvérisation de particules de principe actif en suspension dans une solution de solvant organique et/ou aqueux d'au moins un polymère hydrosoluble et d'au moins un polymère non hydrosoluble sur ces supports sphériques neutres,
- la pulvérisation d'une suspension d'enrobage comprenant au moins une charge inerte en suspension dans la solution d'un mélange d'au moins deux polymères de perméabilités vis-à-vis des milieux gastrique et intestinal différentes sur les particules enrobées obtenues à l'étape précédente,
- éventuellement, le séchage des sphéroïdes médicamenteux ainsi obtenus.

### EXEMPLES

Dans les exemples ci-après, on utilise les excipients pharmaceutiques suivants:
- EUDRAGIT RS 100 : résine polyacrylique-polyméthacrylique peu perméable, commercialisée par la Société RÖHM,
- EUDRAGIT RL 100 : résine polyacrylate-polyméthacrylate perméable, commercialisée par la Société RÖHM,
- PVP K 90: polyvinylpyrrolidone commercialisée par la Société BASF,
- AEROSIL : gel de silice commercialisé par la Société DE-GUSSA,
- Alcool pour usage pharmaceutique,
- Talc pour usage pharmaceutique,
- Phtalate d'éthyle commercialisé par la Société SOLVAY,
- Supports neutres sphériques constitués de saccharose et d'amidon commercialisés par la Société WERNER's,
- INWITOR 900 : monostéarate de glycérol commercialisé par la Société UHLS,
- EUDRAGIT L100 : résine à base d'acide méthacrylique et de méthyl méthacrylate commercialisé par la Société RÖHM.

### Exemple 1 :

### Préparation de sphéroïdes médicamenteux, constitutifs de la forme pharmaceutique selon l'invention et dont le principe actif est le Diltiazem.

Dans un réacteur à lit d'air fluidisé de type GPCG1, on introduit 0,7kg de supports sphériques neutres de 300 à 400µm de diamètre sur lesquels on pulvérise une suspension ayant la composition suivante:

| | |
|---|---|
| EUDRAGIT RS 100 | 0,525kg |
| PVP K 90 | 0,525kg |
| Diltiazem | 7,000kg |
| Alcool pour usage pharmaceutique | 18,760kg |

Puis, on pulvérise sur les particules ainsi formées une suspension d'enrobage ayant la composition suivante:

| | |
|---|---|
| EUDRAGIT RS 100 | 2,000kg |
| EUDRAGIT RL 100 | 0,100kg |
| Phtalate d'éthyle | 0,192kg |
| AEROSIL | 0,320kg |
| Talc pour usage pharmaceutique | 0,400kg |
| Alcool pour usage pharmaceutique | 10,500kg |
| Acétone | 4,500kg. |

On introduit une quantité de 400 à 500 des sphéroïdes médicamenteux obtenus dans des capsules dures de gélatine de constitution classique.

Un essai de libération du principe actif in vitro est réalisé de la façon suivante:

On utilise un dissolumètre conventionnel équipé de palettes ; on utilise de l'eau purifiée comme milieu de dissolution ; les conditions d'utilisation du dissolumètre sont de 900 ml d'eau purifiée et de 100 t/mn ; la quantité de capsules introduites correspond à une unité de prise thérapeutique.

On mesure la quantité de principe actif libéré (exprimée en pourcentage en poids) par rapport à la quantité totale de principe actif comporté par les capsules introduites, de la façon suivante :

On effectue des prélèvements de solution, de 0 à 4 heures, toutes les heures, puis de 4 à 16 heures toutes les deux heures. Le Diltiazem libéré dans les solutions prélevées est dosé au moyen d'un appareil de CLHP muni d'un détecteur UV à 240 nm.

Les résultats obtenus sont rassemblés dans le Tableau 1.

**Tableau 1 :**

| HEURES | % DISSOUS |
|---|---|
| 0 | 0 |
| 1 | 0,8 |
| 2 | 2,0 |
| 3 | 3,4 |
| 4 | 5,1 |
| 6 | 11,7 |
| 8 | 40,4 |
| 10 | 88,0 |
| 12 | 99,4 |
| 14 | 100,9 |

### Exemple 2 :

### Préparation de sphéroïdes médicamenteux, constitutifs de la forme pharmaceutique selon l'invention et dont le principe actif est le Diltiazem.

On prépare des sphéroïdes médicamenteux comme dans l'exemple 1 ci-dessus en utilisant comme suspension d'enrobage, une suspension ayant la composition suivante :

| | |
|---|---|
| EUDRAGIT RS 100 | 3,060 kg |
| EUDRAGIT RL 100 | 0,340 kg |
| Phtalate d'éthyle | 0,340 kg |
| Inwitor 900 | 0,170 kg |
| Isopropanol | 14,28 kg |
| Acétone | 9,52 kg |

De la même façon que dans l'exemple 1 ci-dessus, on mesure la dissolution. Les résultats sont rassemblés dans le tableau 2 ci-dessous.

**Tableau 2 :**

| HEURES | % DISSOUS |
|---|---|
| 0 | 0 |
| 1 | 2,8 |
| 2 | 6,4 |
| 3 | 11,6 |
| 4 | 18,4 |
| 6 | 32,3 |
| 8 | 49,1 |
| 10 | 68,7 |
| 12 | 85.3 |
| 16 | 103,6 |

### Exemple 3 :

### Préparation de sphéroïdes médicamenteux, constitutifs de la forme pharmaceutique selon l'invention et dont le principe actif est le Diltiazem.

On prépare des sphéroïdes médicamenteux comme dans l'exemple 1 ci-dessus, en utilisant comme suspension d'enrobage, une suspension ayant la composition suivante :

| | |
|---|---|
| EUDRAGIT RS 100 | 3,612 kg |
| EUDRAGIT RL 100 | 0,638 kg |
| Phtalate d'éthyle | 0,425 kg |
| Inwitor 900 | 0,213 kg |
| Isopropanol | 17,850 kg |
| Acétone | 11,900 kg |

De la même façon que dans l'exemple 1, on mesure la dissolution. Les résultats sont rassemblés dans le Tableau 3 ci-dessous.

**Tableau 3 :**

| HEURES | % DISSOUS |
|---|---|
| 0 | 0 |
| 1 | 1,1 |
| 2 2 | 7,2 7,2 |
| 3 | 84,3 |
| 4 | 90 |
| 6 | 92,7 |
| 8 | 94,8 |
| 10 | 94 |
| 12 | 95,1 |
| 14 | 95,1 |
| 16 | 96 |

### Exemple 4 :

### Préparation de sphéroïdes médicamenteux, constitutifs de la forme pharmaceutique selon l'invention et dont le principe actif est le Verapamil.

On prépare des sphéroïdes médicamenteux comme dans l'exemple 1 ci-dessus, en remplaçant le Diltiazem comme principe actif par du Verapamil et en utilisant comme suspension d'enrobage, une suspension ayant la composition suivante :

| | |
|---|---|
| EUDRAGIT RS 100 | 1,650 kg |
| EUDRAGIT RL 100 | 0,087 kg |
| EUDRAGIT L 100 | 0,063 kg |
| Phtalate d'éthyle | 0,165 kg |
| Aerosil | 0,274 kg |
| Talc | 0,343 kg |
| Alcool pour usage pharmaceutique | 9,000 kg |
| Acétone | 3,857 kg |

De la même façon que dans l'exemple 1 ci-dessus, on mesure la dissolution, le Vérapamil libéré étant dosé au moyen d'un spectrophotomètre UV à 278 nm. Les résultats sont rassemblés dans le tableau 4 ci-dessous.

**Tableau 4 :**

| HEURES | % DISSOUS |
|---|---|
| 0 | 0 |
| 1 | 1,9 |
| 2 | 6,1 |
| 3 | 12,3 |
| 4 | 21,2 |
| 6 | 49,6 |
| 8 | 82,1 |
| 10 | 94,7 |
| 12 | 97,4 |
| 14 | 97,8 |
| 16 | 98,6 |

## Revendications

1. Forme pharmaceutique multiparticulaire à libération retardée et pulsée, permettant d'obtenir le début de la mise à disposition du principe actif dans les 4 à 8 heures suivant l'ingestion de ladite forme pharmaceutique, puis la libération progressive de la totalité du principe actif dans les 8 à 20 heures suivantes, **caractérisée par le fait qu'**elle est exempte d'acide organique et qu'elle se présente sous la forme de sphéroïdes médicamenteux constitués d'un support sphérique neutre comportant un premier enrobage à base d'un mélange d'au moins un polymère hydrosoluble et d'au moins un polymère non hydrosoluble au sein duquel sont uniformément réparties des particules constitutives d'un principe actif, l'ensemble comportant un deuxième enrobage à base d'au moins deux polymères pH indépendants, présentant des degrés de perméabilité vis-à-vis des milieux gastrique et intestinal différents, éventuellement d'au moins un polymère pH dépendant, d'au moins un plastifiant et d'au moins une charge inerte uniformément répartie dans ledit enrobage.

2. Forme pharmaceutique selon la revendication 1, **caractérisée par le fait que** le principe actif représente 20 à 100% en poids de la couche d'enrobage entourant le support sphérique neutre, de préférence de 60 à 90% en poids, et plus préférentiellement encore de 80 à 90% en poids.

3. Forme pharmaceutique selon l'une des revendications 1 et 2, **caractérisée par le fait que** le rapport massique entre le polymère hydrosoluble et le polymère non hydrosoluble est de 0,1 à 0,9, de préférence de 0,3 à 0,7, et plus préférentiellement encore de 0,45 à 0,55.

4. Forme pharmaceutique selon l'une quelconque des revendications 1 à 3, **caractérisée par le fait que** le polymère hydrosoluble est choisi dans le groupe comprenant notamment la polyvinylpyrrolidone, l'hydroxypropylméthylcellulose et leurs mélanges, et le polymère non hydrosoluble est choisi dans le groupe comprenant notamment les résines acryliques et/ou méthacryliques, les polymères cellulosiques et leurs mélanges.

5. Forme pharmaceutique selon l'une quelconque des revendications 1 à 4, **caractérisée par le fait que** la proportion entre le polymère présentant la perméabilité la plus faible et le polymère présentant la perméabilité la plus grande est de 97/3 à 80/20.

6. Forme pharmaceutique selon l'une quelconque des revendications 1 à 5, **caractérisée par le fait que** les polymères entrant dans la constitution du deuxième enrobage sont choisis dans le groupe comprenant notamment les résines acryliques et/ou méthacryliques, les polymères cellulosiques, et leurs mélanges.

7. Forme pharmaceutique selon l'une quelconque des revendications 1 à 6, **caractérisée par le fait que** la charge inerte uniformément répartie dans le deuxième enrobage est choisie dans le groupe comprenant notamment le talc, la silice colloïdale anhydre, le stéarate de magnésium, le monostéarate de glycérol et leurs mélanges.

8. Forme pharmaceutique selon l'une quelconque des revendications 1 à 7, **caractérisée par le fait que** le principe actif est choisi dans le groupe comprenant notamment les molécules actives sur le système cardiovasculaire, et plus particulièrement le Diltiazem et le Verapamil, les anti-inflammatoires, les antiallergiques et les antihistaminiques,

9. Procédé de préparation d'une forme pharmaceutique selon l'une quelconque des revendications 1 à 8, **caractérisé par** une succession d'étapes comprenant:
- l'introduction de supports sphériques neutres dans une enceinte réactionnelle à lit fluidisé,
- la pulvérisation de particules de principe actif en suspension dans un solvant organique et/ou aqueux d'au moins un polymère hydrosoluble et d'au moins un polymère non hydrosoluble sur ces supports sphériques neutres,
- la pulvérisation d'une suspension d'enrobage, comprenant au moins une charge inerte en suspension dans la solution d'un mélange d'au moins deux polymères de perméabilités différentes, vis-à-vis des milieux gastrique et intestinal sur les particules enrobées obtenues à l'étape précédente,
- éventuellement, le séchage des sphéroïdes médicamenteux ainsi obtenus.

## Patentansprüche

1. Multipartikuläre pharmazeutische Form mit verzögerter und gepulster Freisetzung, die es ermöglicht, den Beginn der Verfügbarkeit des Wirkstoffes in 4 bis 8 Stunden nach der Einnahme der genannten pharmazeutischen Form zu erhalten, woran sich die schrittweise Freisetzung der Gesamtheit des Wirkstoffes in den folgenden 8 bis 20 Stunden anschließt, **dadurch gekennzeichnet, daß** sie frei von organischer Säure ist und daß sie in Form von Arzneimittel-Kügelchen vorliegt, bestehend aus einem neutralen kugelförmigen Träger, der eine erste Umhüllung auf der Basis einer Mischung von mindestens einem wasserlöslichen Polymer und mindestens einem nicht wasserlöslichen Polymer umfaßt, worin die im wesentlichen aus einem Wirkstoff bestehenden Teilchen gleichmäßig verteilt sind, wobei das Ganze eine zweite Umhüllung auf der Basis von mindestens zwei vom pH-Wert unabhängigen Polymeren, die gegenüber dem gastrischen und intestinalen Milieu unterschiedliche Permeabilitäts-Grade aufweisen, sowie gegebenenfalls mindestens ein vom pH-Wert abhängiges Polymer, mindestens einen Weichmacher und mindestens einen inerten Füllstoff umfaßt, der in der genannten Umhüllung gleichmäßig verteilt ist.

2. Pharmazeutische Form nach Anspruch 1, **dadurch gekennzeichnet, daß** der Wirkstoff 20 Gew.-% bis 100 Gew.-%, vorzugsweise 60 Gew.-% bis 90 Gew.-% und noch bevorzugter 80 Gew.-% bis 90 Gew.-% der Umhüllungsschicht darstellt, die den neutralen kugelförmigen Träger umgibt.

3. Pharmazeutische Form nach einem der Ansprüche 1 und 2, **dadurch gekennzeichnet, daß** das Massenverhältnis zwischen dem wasserlöslichen Polymer und dem nicht wasserlöslichen Polymer von 0,1 bis 0,9, vorzugsweise von 0,3 bis 0,7 und noch bevorzugter von 0,45 bis 0,55 beträgt.

4. Pharmazeutische Form nach irgendeinem der Ansprüche 1 bis 3, **dadurch gekennzeichnet, daß** das wasserlösliche Polymer aus der Gruppe gewählt wird, die insbesondere Polyvinylpyrrolidon, Hydroxypropylmethylcellulose und ihre Mischungen umfaßt, und das nicht wasserlösliche Polymer aus der Gruppe gewählt wird, die insbesondere die Acrylharze und/oder Methacrylharze, die Cellulosepolymere und ihre Mischungen umfaßt.

5. Pharmazeutische Form nach irgendeinem der Ansprüche 1 bis 4, **dadurch gekennzeichnet, daß** das Verhältnis zwischen dem Polymer, das die geringste Permeabilität aufweist, und dem Polymer, das die größte Permeabilität aufweist, von 97/3 bis 80/20 beträgt.

6. Pharmazeutische Form nach irgendeinem der Ansprüche 1 bis 5, **dadurch gekennzeichnet, daß** die Polymere, die in die Struktur der zweiten Umhüllung eintreten, aus der Gruppe gewählt werden, die insbesondere die Acrylharze und/oder Methacrylharze, die Cellulosepolymere und ihre Mischungen umfaßt.

7. Pharmazeutische Form nach irgendeinem der Ansprüche 1 bis 6, **dadurch gekennzeichnet, daß** der in der zweiten Umhüllung gleichmäßig verteilte inerte Füllstoff aus der Gruppe gewählt wird, die insbesondere Talk, wasserfreie kolloidale Kieselerde, Magnesiumstearat, Glycerinmonostearat und ihre Mischungen umfaßt.

8. Pharmazeutische Form nach irgendeinem der Ansprüche 1 bis 7, **dadurch gekennzeichnet, daß** der Wirkstoff aus der Gruppe gewählt wird, die insbesondere gegenüber dem cardiovasculären System aktive Moleküle und ganz besonders Diltiazem und Verapamil, die anti-inflammatorischen Mittel, die Antiallergene und die Antihistaminika umfaßt.

9. Verfahren zur Herstellung einer pharmazeutischen Form nach irgendeinem der Ansprüche 1 bis 8, **gekennzeichnet durch** eine Aufeinanderfolge von Stufen, die umfaßt:
- Eintragen von neutralen kugelförmigen Trägern in einen Reaktionsbehälter mit fluidisiertem Bett,
- Versprühen der Wirkstoff-Teilchen in Suspension eines organischen und/oder wäßrigen Lösungsmittels, von mindestens einem wasserlöslichen Polymer und mindestens einem nicht wasserlöslichen Polymer auf diese neutralen kugelförmigen Träger,
- Versprühen einer Umhüllungs-Suspension, die mindestens einen inerten Füllstoff in Suspension in der Lösung einer Mischung von mindestens zwei Polymeren mit gegenüber dem gastrischen und intestinalen Milieu unterschiedlicher Permeabilität umfaßt, auf diese in der vorstehenden Stufe erhaltenen umhüllten Teilchen,
- gegebenenfalls Trocknen der auf diese Weise erhaltenen Arzneimittel-Kügelchen.

## Claims

1. Multiparticulate pharmaceutical form with delayed and pulsed release, enabling to obtain the onset of the availability of the active ingredient within 4 to 8 hours after the ingestion of the pharmaceutical form, and then a progressive release of the totality of the active ingredient within the 8 to 20 following hours, wherein it is free of organic acid and that it is in the form of medicinal spheroids consisting of a neutral spherical core comprising a first coating based on a mixture of at least one hydrosoluble polymer and of at least one non hydrosoluble polymer throughout which are uniformly distributed the constitutive particles of an active ingredient, the whole comprising a second coating based on at least two pH independent polymers presenting rates of permeability different from one another with respect to the gastric and intestinal mediums, optionally at least one pH dependent polymer, at least one plasticizer and at least one inert carrier uniformly distributed throughout the said coating.

2. Pharmaceutical form according to claim 1, wherein the active ingredient represents from 20 to 100%, preferably from 60 to 90%, and more preferably from 80 to 90%, by weight of the coating which envelops the neutral spherical core.

3. Pharmaceutical form according to claim 1 or claim 2, wherein the weight ratio between the hydrosoluble polymer and the non hydrosoluble polymer is from 0.1 to 0.9, preferably from 0.3 to 0.7, and more preferably from 0.45 to 0.55.

4. Pharmaceutical form according to any one of claims 1 to 3, wherein the hydrosoluble polymer is selected from the group comprising especially polyvinylpyrrolidone, hydroxy-propylmethyl cellulose and their mixtures, and the non hydrosoluble polymer is selected from the group comprising especially acrylic and/or methacrylic resins, cellulosic polymers and their mixtures.

5. Pharmaceutical form according to any one of claims 1 to 4, wherein the proportion between the polymer which presents the lowest permeability and the polymer which presents the highest permeability, is from 97/3 to 80/20.

6. Pharmaceutical form according to any one of claims 1 to 5, wherein the polymers of the second coating are selected from the group comprising especially acrylic and/or methacrylic resins, cellulosic polymers and their mixtures.

7. Pharmaceutical form according to any one of claims 1 to 6, wherein the inert carrier which is uniformly distributed throughout the second coating is selected from the group comprising especially talc, anhydrous colloidal silicone dioxide, magnesium stearate, monostearate of glycerol and their mixtures.

8. Pharmaceutical form according to any one of claims 1 to 7, wherein the active ingredient is selected from the group comprising especially the molecules which are active on the cardio-vascular system, and more especially Diltiazem and Verapamil, the anti-inflammatories, the antiallergics and the antihistamines.

9. Process for the preparation of the pharmaceutical form according to one of claims 1 to 8, comprising:
- the introduction of neutral spherical cores into a reaction vessel working on the principle of the fluidised-bed,
- the spraying onto the said neutral spherical cores of particles of the active ingredient suspended in a solution of at least one hydrosoluble polymer and of at least one non hydrosoluble polymer in an organic and/or aqueous solvent,
- the spraying on the coated particles obtained in the preceding step of a coating suspension comprising at least one inert carrier suspended in a solution of a mixture of at least two polymers having permeabilities different from one another with respect to the gastric and the intestinal mediums.
- optionally the drying of the medicinal spheroids thus obtained.
